# EUROPEAN PATENT APPLICATION

(11) **EP 1 110 523 A1**
(43) Date of publication of application: **27.06.2001**
(21) Application number: 99125931.8
(22) Date of filing: 23.12.1999
(51) Int. Cl.: A61F 13/15, A61F 5/455

(54) **Liquid handling systems comprising three-dimensionally shaped membranes**

(71) Applicant: THE PROCTER & GAMBLE COMPANY, Cincinnati, Ohio 45202 (US)
(72) Inventor: Schmidt, Matthias, 65510 Idstein (DE); Ehrnsperger, Bruno Johannes, 65936 Frankfurt (DE)
(74) Representative: Kohol, Sonia

(57) **Abstract**

A liquid handling member such as for application in hygiene articles, which comprises a membrane assembly separating a first and a second zone, which is connected to a suction device. This assembly comprises a membrane material having a actual surface area along its surface contours, and also has a projected surface area correspoding to an area projected to surface generally aligned with the member surface during its intended use.

## Description

These present invention relates to hygiene articles, in particular to articles intended to receive bodily liquids, such as urine in case of absorbent articles like diapers or external or internal catheter, or menses or blood, such as for feminine protection articles or wound treating articles, sweat, saliva.

### Background / Prior art

Liquid handling systems comprising a membrane are known in the art. For example, US-A-5.678.564 discloses a liquid removal system designed to permit liquid removal through the use of an interface device. The interface device is provided with a membrane which has and is capable of maintaining a vacuum on one side so that when liquid contacts the opposite side of the membrane the liquid passes through the membrane and is removed from the interface device by a maintained vacuum to a receptacle for disposal. Such a system is described to be useful as a female external catheter system. Also, in PCT application US99/14654 the possibility of increasing the effective surface area and of corrugating the membrane is described.

Whilst corrugations, as well as other ways to increase the effective surface area are well known for other applications, none of the prior art hitherto identified the appropriate design criteria for useful increase of effective surface area for absorbent article with liquid handling systems comprising a membrane material.

Henceforth, the present invention aims at overcoming the limitation of prior art liquid handling devices by providing devices having suitable membrane designs with an effective surface area increase defined by appropriate design criteria.

In another aspect, the present invention relates to the method of making a liquid handling member, wherein a membrane material is submitted to a morphology change, such as being corrugated, which is then fixed.

Such liquid handling members are particularly useful for applications in the hygiene field, such as hygienic articles like external catheter.

### Summary

The present invention is a liquid handling member comprising an first zone, and a second zone connected to a suction device, wherein first zone and second zone are separated by a porous membrane assembly. This assembly comprises a membrane material having a actual surface area along its surface contours, and also has a projected surface area correspoding to an area projected to surface generally aligned with the member surface during its intended use.

The membrane assembly of the absorbent memebr can be described by having two enveloping surfaces generally parallel to the projected surface area, whereby the membrane assembly is capable of maintaining a pressure differential between the second zone and the first zone without permitting air to penetrate from the first zone to the second zone. Further, the membrane material in this assembly has an actual surface area which is at least 2, preferably 8, more preferably 20, even more preferably 40 times the area of the projected surface of the membrane assembly, and which is not more than 200, preferably not more than 100, and even more preferably not more than 80 times the area of the projected surface of the membrane assembly, when measured without a load applied to the member, but preferably also when measured under an external load pressure of at least about 2070 Pa (0.3 psi), preferably when submitted to a pressure exceeding about 4800 Pa (0.7 psi), or even about 9650 Pa (1.4 psi), when applied perpendicular to the projected surface of said membrane assembly.

When describing a liquid handling member according to the present invention by using a local Cartesian coordinate system with a x (length), y (width), and z (thickness) direction, the two enveloping surfaces are arranged at a distance H from each other, which should be greater than the material thickness of the membrane material, which is generally aligned with the flow path of the liquid penetrating through the pores of the membrane material.

Suitable membrane assemblies can have a three-dimensionally shaped morphology having repeating geometric cells defined by repeating geometric pattern of cross-sectional view through the membrane assembly, which can be arranged in a checkerboard pattern or, a row pattern.

In a particular embodiment, the repeating geometric cells are in the form of corrugations, pleats, or folds of a sheet-like membrane material having a pore size diameter r and a material sheet thickness d, which further can be arrranged in by having more than 0.3 corrugation, pleats or folds per centimeter, or by having less than 20 corrugation, pleats or folds per centimeter. The height of these corrugation, pleats or folds having a is preferably more than 0.05 mm and less than 30 mm. The corrugations, pleats or folds can have a repeating cross-sectional pattern, which can be circular, sinusoidal, parabolic, elliptic. These geometric cells have a characteristic height H, and repeating unit width L, wherein the ratio (L^2/H) is at least 10 times, preferably 20 time, more preferably 50 times the ratio of (r^2/d), without a pressure applied, and preferably even under an external load pressure of at least about 2070 Pa (0.3 psi), preferably when submitted to a pressure exceeding about 4800 Pa (0.7 psi), or even about 9650 Pa (1.4 psi), when applied perpendicular to the projected surface of the membrane assembly and related to the projected surface area.

A particular embodiment of the present invention is a liquid handling member wherein the membrane assembly further comprises a support structure for maintaining the geometric shape, which can be alinged with the enveloping surface of the membrane assembly, and then should have an open permeablity structure such as by having liquid permeability of 1000 times preferably 100.000 times the permeability of the membrane.

When the membrane assembly is corrugated, pleated of folded, the support structure can be arranged to fix the corrugations, pleats or folds, and can be affixed by a fixation means, preferably by adhesive or melt fusion, potentially with a non-continuous bonding pattern, preferably point bonding.

The support structure can comprise elastomeric materials, such as sheet material, such as a net, scrim, woven, knitted, or nonwoven material or a film. The support structure can also be in the form of bands, or strands or struts, and can have non-isotropic elastic behaviour.

In a preferred embodiment, the liquid handling member according to the present invention is soft or deformable, and has a buckling force as measured according to the Bulk Softness method of less than 10 N, preferably less than 5 N or more preferably even less than 3 N, and most preferably less than 1 N.

In a further aspect, the present invention is the process of making liquid handling members, with the steps of providing a porous membrane material having a bubble point pressure, creating a morphology change of the membrane material so as to increase the projected versus actual surface for the membrane region; and fixing the morphology change to form a membrane assembly. Further included can be the steps of attaching and hermetically sealing the membrane assembly to a suction device, such that the membrane material separates a first zone (outside of the member) from a second zone inside the member, which is connected to the suction device, such that for a pressure differential between the first and the second zones, which is below the bubble point pressure of the membrane material, liquid can penetrate through the membrane but gas not. Therein, the morphology change can be a selective removal of membrane material from a precurse membrane material, or can be a plastic deformation of a plastically deformable membrane material.

When the membrane material is an essentially two-dimensional sheet material, this can be deformed by stretching, such as tentering, nip roll stretching with varying roll speeds, or by feeding the material between to intermeshing rolls, or by hydroforming, or vacuum forming, or blow molding. The morphology change can also be achieved by corrugating, pleating or folding the membrane material, and the morphology change can be fixed by attaching a support material to the membrane material.

Thereby the support material can be attached in a stretched state to a non-stretched membrane material, such that upon release of the stretch corrugation, pleats or folds are formed.

### Brief description of drawings

- Fig. 1a:: Perspective view of a flat membrane;
- Fig. 1b:: Perspective view of such a material with corrugations;
- Fig. 2:: Schematic cross-sectional view through a rectangularly folded membrane;
- Fig.3:: Perspective view of membrane with protuberances extending in both directions of the surface.
- Fig. 4 -: Softness test sample holder

### Detailed description

In the context of the present invention, a "liquid handling member" is considered to be a device, wherein a liquid is penetrating through a membrane by a driving force such as a suction like a vacuum. If this member is connected to a liquid delivery source, or liquid receiving sink, thus forming a "liquid handling system", this can be used in applications such as for - but without being limited to - receiving body liquids. In such applications, the liquid to be transported will be generally water based, such as body liquids like urine. It will be apparent to the skilled person, that the present invention is not limited to such applications, but that it can be readily re-applied to other liquids such as oily substances as disclosed in PCT applications US 99/14644 or US 99/14645.

Such systems function by the principle that certain porous membranes under certain conditions can be permeable to liquids, but not to gases like air, as long as the "potential differential " such as the pressure differential between the two sides of such a membrane does not exceed a certain value, which is characteristic for a given material and given liquid in the pores of the material - the "bubble point pressure". This latter is often expressed in "height of water column" which corresponds to the pressure exerted by such a column on the material under normal gravity conditions.

For aqueous liquids, the material for the membrane is preferably hydrophilic and has a pore size on the order of about 5 to about 30 µm, more preferably about 10 to 20 µm. Once the membrane has been wetted it will support a suction pressure typically corresponding to about 12.5 cm to about 150 cm height of a water column without permitting air to pass therethrough. Thus, if suction is applied to one side of a wetted membrane, liquid contacting the membrane on the other side will be drawn by the suction through the membrane to the other side of the membrane, from where it can further be removed, for example by being sucked by means of a vacuum through a drain tube to a reservoir. As long as the filter material or membrane remains wet, air does not pass through the filter and suction is maintained without active pumping. If too much suction (too high vacuum) is applied to the membrane there is a risk that the bubble point of the membrane will be surpassed and there will be no liquid in one or more pores of the membrane, thereby allowing air or gas to penetrate through, which can lead to a loss of the vacuum, and of the liquid handling functionality. Thus the amount of vacuum should approach as close as possible - but not exceed - the bubble point.

Thereby, the membrane needs to maintain a certain degree of wetness, so as to maintain the pores filled with liquid, even under suction vacuum, and/or evaporation conditions. As described for example in US-A-5.678.564, the membrane material can be prewetted during manufacture. This may be done by any suitable liquid having preferably a low vapor pressure. Glycerin has been proposed as a prewetting agent because it has a significant smaller propensity for drying out by evaporation and thus can support the vacuum until the first wetting during use.

Generally, such systems can exhibit relatively high liquid transport rates. Thereby, it has been found useful to consider liquid "flux" through the system, expressed in flow of liquid per unit area of the system, e.g. [ml/sec/m²].

However, whilst such systems do exhibit high fluxes compared to other systems, there is still the inherent limitation of the system, namely the need to balance permeability and bubble point pressure - i.e. if a certain bubble point pressure needs to be maintained, the permeability of the membrane cannot be increased ad libitum. This is based on the fact, that the surface area specific flow rate through such a membrane is determined by the surface energies of the membrane material in relation to the liquid, and the liquid permeability of the material. The driving pressure cannot be increased in such designs beyond the bubble point pressure of the membrane, as otherwise the membrane would loose its gas impermeability. Consequently, the maximum flow rate is fixed by the choice of membrane materials, type of liquid, and the differential pressure (not exceeding the bubble point pressure) and the available surface area. This limitation becomes particularly relevant for certain applications, such as in hygienic articles such as external catheters, where it can be desired to handle large flow rates, combined with the desire to have small articles, so as to increase comfort of the wearer.

Henceforth, as already been described in general terms e.g. in PCT application US99/14654, an increase in effective surface area is desirable, such as by corrugating the membrane. However, it has now been found, that increasing the surface area by corrugation not necessarily leads to an improved performance, but under certain conditions can lead to a reduction of performance. Thus, it has been found, that a careful balancing of various parameter needs to be considered when designing liquid handling members or systems.

Thus, it is a goal to maximize the flow by increasing the "effective surface area" of the membrane, without increasing the effective in-use surface area or "projected surface area", thereby still allowing small design dimension of the total system.

In the context of the present invention, a distinction will be made between a "membrane material", which is essentially defined by having membrane functionality, i.e. being permeable to liquids but not to gases up to the bubble point pressure of the material, and a "membrane assembly", wherein such a membrane material has a particular morphology or is arranged in a particular way, such as by being corrugated, optionally combined with other materials, such as by comprising a "support material" as discussed hereinafter.

Further, the term "projected surface area" relates to the area, which a viewer would identify on a macroscopic view when looking at the membrane assembly. For example, if such a system would be included in an external catheter, the projected surface area corresponds to an orthogonal projection of the surface area of the membrane assembly to a plane corresponding to the relevant surface of the external catheter when positioned on the wearer. In many instances, this projection can be done without too large of an error by projecting it to a plain plane, such as image analysis systems do. Accordingly, the "projected surface area" in a flat article, such as a conventional baby diaper with primarily width and length (x-y)-extensions at a relatively small thickness (z-direction) extension, could be determined by positioning such an article flat on a horizontal plane, and thus determine the orthogonal projection to this flat surface.

The "actual surface area" of the membrane material in this membrane assembly is considered to correspond to the overall surface of the membrane material, however, not considering the "inner surface" of the membrane, which corresponds to the pores within the membrane material. For example, for an essentially flat membrane made of a porous material, this "actual surface area" becomes identical to the projected surface, whilst the total surface such as would be determined by known techniques such as BET determination with nitrogen as adsorption gas, would be larger by also including the "inner surface" of the membrane, i.e. the surface of the pores within the material.

When the pores in the membrane material do not have a constant diameter, e.g. considering a cross-section through such a pore having the shape of a cone, the wide part of the pore (respectively of the cone) can be considered to belong to the actual available surface, whilst the smaller parts of the cone, with diameters less than the one corresponding to the bubble point pressure, would be considered to belong to the "inner" surface. When not considering circular pore cross-section, the above discussion considers the cross-sectional equivalent diameter.

A further useful term is the "effective area ratio" defined as the ratio of the "actual surface area" to the "projected surface area", which equals to one for a flat membrane, and will desirably be higher, preferably have values of at least 2, preferably at least 8, more preferably 20 and even more preferably more than 40. However, it has been found that a too high value for this ratio is not desired, as then the liquid transport through the membrane assembly is not increased any more, but actually can - with an increase of this ratio actually descreases. Henceforth, this ratio should not exceed the value of 200, preferably of 100, and more preferably less than 80.

When considering various applications of such members, these effective area ratios should not only be achieved when being produced, but should withstand applied pressures such as during manufacturing, storage, but also during use. In particular, it is preferred, that these ratios are maintained when the liquid transport member is submitted to an applied load exerting a pressure perpendicular to the projected surface of at least about 2070 Pa (0.3 psi), preferably when submitted to a pressure exceeding about 4800 Pa (0.7 psi), or even about 9650 Pa (1.4 psi).

Generally, the membrane materials will exhibit a length and width dimension, such as can be expressed in orthogonal x, and y- coordinates, and also have a thickness dimension, corresponding to the orthogonal z-direction. Typically, sheets will have x-, and y-dimensions significantly exceeding the z (or thickness) dimensions. Such flat structures can be arranged in 3D-shape - such as by folding or corrugating or waving the sheet, such that the resulting structure (the membrane assembly) could (again) be seen having a z-dimension now significantly increased over the thickness of the original sheet but still be significantly exceeded by the x-, and y- dimension. For clarification, the well-known corrugated card-board has a layer of thin material corrugated and laminated to other thin layers, whilst the composite still forms a sheet-like material.

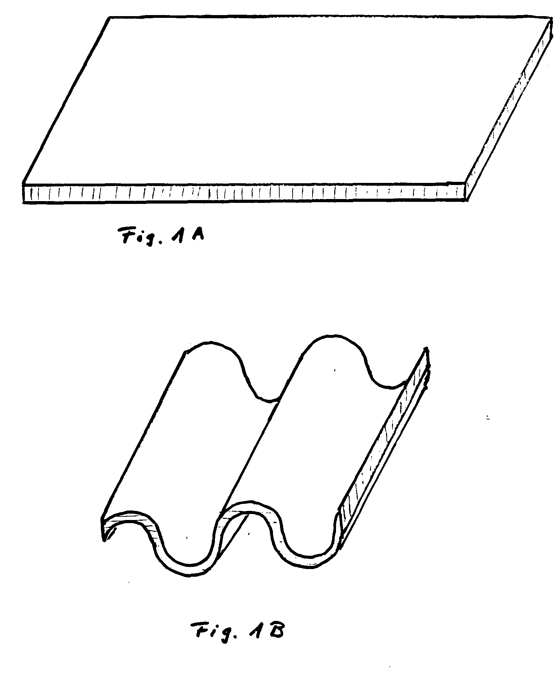

In order to further explain the present invention, reference is first made to one particular execution for a membrane assembly useful for liquid handling members, namely to an increase of the "effective area ratio" by corrugating, pleating or folding an otherwise essentially flat or sheet-like membrane material, i.e. which has width and length ( x-, and y) - dimensions being much larger than the thickness or z-dimension (refer to Fig. 1a and 1b for a schematic comparison).

Corrugations are geometrical structures, having vales and crests arranged in a repeating, generally parallel arrangement, wherein the cross-section can be described by repeating units of rectangles, triangles, sinusoidal curves, circular segments, or the like. Of course, the overall article can comprise several corrugated regions, whereby the corrugations of each of these regions do not need to be the same.

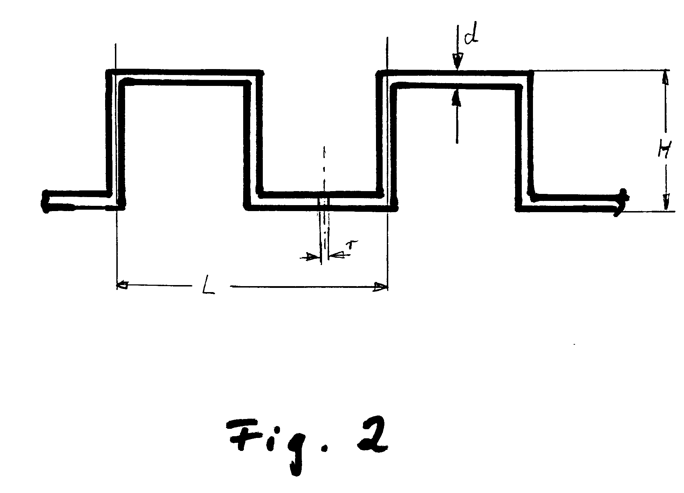

In figure 2, an example of a cross-sectional view through such a pattern is schematically depicted, now approximated by a membrane assembly having a repeating rectangular geometric units. The characteristic dimensions of these units are the height "H", and the length "L" (in wave-shaped corrugations, this corresponds to the double of the amplitude and to the wavelength). The membrane material further has a thickness "d" and a pore radius "r", both assumed for the following discussion to be constant throughout the membrane material.

Surprisingly, is has now been found, that increasing the effective area ratio beyond certain limits can result in poorer performance of the member.

Without wishing to be bound by the theory, and explained for the exemplary structure of a corrugated assembly, it is believed, that an increase in corrugation surface improves the overall liquid handling performance, as long as the "permeability" of the corrugations is sufficiently high when compared to the permeability of the membrane material. As explained in more detail in PCT application US99/14654, the latter is a function of the square of the membrane pore size divided by the membrane thickness (e.g. both measured in µm). The effect of the corrugation on the total permeability is depending on the characteristic dimensions of the corrugations, such that the ratio of the square of the corrugation length "L" divided by the corrugation height "H". Henceforth, it has been found desirable to use designs wherein the ratio L²/ H is high when compared to the ratio of r²/ d of the membrane - thus the first ratio a should be at least 5 times, preferably at least 10 times, more preferably at least 20 times or even 100 times the second ratio.

Thus, for a given membrane material and either a given height of width dimension of the corrugation, the corresponding other dimension can be determined. Similarly, for a given membrane material, suitable L and H ratios can readily determined to provide a material with maximized liquid handling capability.

Preferably these ratios are maintained also for the already described pressure ranges, namely for an applied load exerting a pressure perpendicular to the projected surface of at least about 2070 Pa (0.3 psi), preferably when submitted to a pressure exceeding about 4800 Pa (0.7 psi), or even about 9650 Pa (1.4 psi), whereby the pressure is related to the projected surface area on the membrane assembly.

When considering repeating geometric units of rectangular shape of corrugations (Fig. 2), the height and width of the corrugation can readily be determined. When considering corrugations with differently shaped cross-sections, or pleats or folds, the characteristic length is being defined by the distance of one repeating unit, and the height by the maximum extension from the "base line" the latter being defined as the overall circumscribing curve.

For aqueous liquid handling members, with membrane materials having typical pore sizes of 5 to 50 µm and typical thickness of more than 5 µm to 100 µm, typical limits for the respective corrugation dimensions are more than 0.3 corrugations per centimeter, but less than 20 per cm, corresponding to a "Corrugation unit length" of more than 0.05 mm and less than 35 mm, and more than 0.05 mm but less than 30mm for the height of the corrugations.

In case of more complex cross-sectional shapes, the height dimension is defined as the maximum fluid path from the "bulk of the liquid" to the most remote corner of the corrugation. In case of a membrane assembly comprising different regions with different patterns, the above will be applied to each of these regions. In case of a membrane assembly having a non-repeating or random corrugation pattern, the characteristic dimensions can be averaged over the respective area.

A more general way to describe the effect of the corrugation is by comparing the ratio of permeability to the membrane assembly thickness, and to plot this for various corrugation patterns. For example, if the height of the corrugations is kept constant, and the width of the corrugations is reduced, the permeability/thickness ratio will first increase because of an increase in available area. As of the critical area ratio, however, the permeability to thickness ratio will not increase any more, and even decrease - because of the corrugations being too close to each other or too deep, thereby limiting the flow. The permeability to thickness ratio measurement is described in already referred to PCT application US99/14654.

Whilst the above explanation was primarily directed to a simple corrugated structure, there are a number of other structures, which are useful for the present invention. The basic principle for such structures is, that they show an increase of effective surface area, without unduly limiting the permeability of the resulting structure and/or without unduly increasing the thickness of the structure. Provided the above requirements are satisfied, the corrugated membrane can be corrugated again, i.e. if for example first, small corrugations are created in an overall flat structure, this structure can be corrugated in a secondary corrugation which is larger than the first. In following fractale geometry considerations, this can be continued to create assemblies of multiple corrugations. The structure can have - at least in parts thereof - a regular, repeating pattern, or the structure can have an irregular shape. Then the appropriate characteristic dimensions as used in the above determination will be applied to an appropriately chosen sub-region, and averaged thereover by conventional mathematical calculations.

In general terms, such structures can be described by having two characteristic geometric parameter - first the primary thickness corresponding to the effective "pore length" of the membrane material (i.e. the length of the shortest path for a liquid element to pass through the membrane material), and second the apparent thickness of the membrane assembly such as can be determined by considering a surface which geometrically envelops the membrane assembly. For example, for flat corrugated structures, such an envelop would be represented by two plain surfaces connecting the ridges of the corrugations on both sides of the membrane assembly. For more irregular surfaces, the skilled person will be able to readily determine this envelope according to conventional considerations. If, for example a secondary corrugation structure would be considered, the envelop would smoothly connect the ridges of the larger corrugations, but not of the smaller ones.

A structure according to the present invention then has membrane assembly thickness defined between these enveloping surfaces, which is larger than the thickness of the membrane material along the flow path of the liquid through the membrane material.

If membrane assemblies are created whereby a certain void region becomes disconnected from the enveloping surface, e.g., by forming an open space surrounded by membrane material, this would not be considered to contribute to the available surface area of the membrane, but would be considered like an inner pore of the membrane material (without, however, having a detrimental effect on the bubble point pressure).

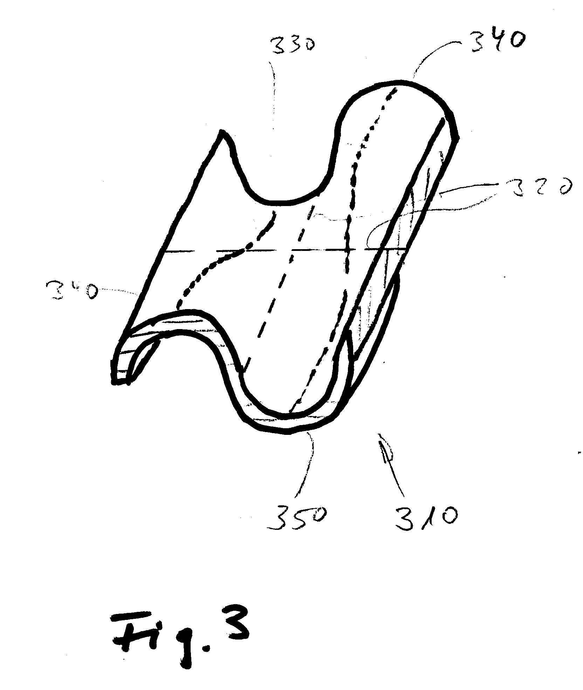

When the membrane assembly according to the present invention has a morphology with a regularly repeating pattern, this can be of the type as explained for corrugations, i.e. having ridges and vales extending in one direction, and having a repeating geometric unit extending in the other two dimensions - such as the rectangular pattern as depicted in Fig.1. Alternatively, the repeating unit can be a three-dimensional geometric unit, such as exemplified in Fig. 3, showing a "checkerboard" type structure with protuberances extending into both directions in the various sections. Therein, the geometric unit is depicted with protuberances 340 extending upwardly (relative to the direction of the viewer), and protuberances 350 extending downwardly. The connecting lines 330 of the ridges are curvelinear, whilst therein the centerlines 320 are shown as straight lines. Of course, these geometric repeating units need not to be in a perpendicular relation, or can have varying extensions into the various directions, or can be non-rectangular.

A membrane assembly according to the present invention can also essentially consist of membrane materials which have a morphology with a regularly repeating geometric pattern without being formed from a sheet like material. Such a structure can be three-dimensionally shaped porous materials (such as a sponge or a foam) with crests and valleys therein. As for the corrugated structures, fractale geometry can create sub-patterns for such crests and valleys, thereby increasing the effective surface area up to the upper limitations as described hereinafter.

A useful structure can also be made of an apertured film material, with a macroscopic surface of the material having a 3D structure, such as can be achieved by methods as described hereinafter. Such a structure can be a film-like membrane, which has macroscopic indentations or protuberances, either all into one direction or some extending away from the opposite surface of the structures.

If - as for some of the processes as described hereinafter - the increase of the membrane assembly surface results in a modification of the pore size of the membrane material used therein, it should be noted, that the respective liquid handling requirements should be met by the membrane properties after this has undergone this process. In a preferred aspect, the bubble point pressure of such a modified material should be close to the bubble point pressure of the original membrane material more preferably be not less than 90% of the bubble point pressure value of the latter.

In addition to the above described fluid handling properties, the membrane assemblies should satisfy various other requirements with regard to usefulness for the intended use, in particular absorbent articles, such as hygiene articles, and especially external catheter. Henceforth, the materials should be compatible with the skin of a wearer, and not be unduly stiff, to also comply with the body contours of the wearer, and or to comply to change of the body contours during use, such as by movements or change of position.

Such softness provides increased comfort during wear. As is well known softness is a subjective, multi-faceted property including components such as bending resistance, buckling resistance and coefficient of friction. As is also known the tensile properties of a material are also important as a predictor of softness. In particular, materials having a low tensile modulus and high elongation are desirable.

Bending and buckling resistance are particularly important properties. However, as also well known from corrugated cardboards, the bending in x- or y- direction (i.e perpendicular to the thickness direction) can be impacted by the particular surface morphology.

An especially desirable measure of the bending component of softness in the case of absorbent article core components has been found to be buckling resistance. As will be recognized by one of skill in the art, the corrugated structure as described in the above can assume an arcuate configuration during use. The Bulk Softness test described in the Test Methods section below uses resistance to compressive deformation of a sample having a controlled arcuate configuration as a measure of the softness of the sample. Suitably, structure according to the present invention has a buckling force of less than about 10 Newtons. Preferably, the buckling force is less than about 5 Newtons, more preferably, less than about 3 Newton, and most preferably 1 Newton.

Suitable materials can be open celled foams, such as High Internal Phase Emulsion foams, can be Cellulose Nitrate Membranes, Cellulose Acetate Membranes, Polyvinyldifluorid films, non-wovens, woven materials such as meshes made from metal, or polymers as m Polyamide, or Polyester. Other suitable materials can be apertured Films, such as vacuum formed, hydroapertured, mechanically or Laser apertured, or films treated by electron, ion or heavy-ion beams.

Specific materials are Cellulose acetate membranes, such as also disclosed in US 5,108,383 (White, Allied-Signal Inc.), Nitrocellulose membranes such as available from e.g. from Advanced Microdevices (PVT) LTD, Ambala Cantt. INDIA called CNJ-10 (Lot # F 030328) and CNJ-20 (Lot # F 024248)., Cellulose acetat membranes, Cellulose nitrate membranes, PTFE membranes, Polyamide membranes, Polyester membranes as available e.g. from Sartorius in Göttingen, Germany and Millipore in Bedford USA, can be very suitable. Also microporous films, such as PE/PP film filled with CaCO₃ particles, or filler containing PET films as disclosed in EP-A-0.451.797.

Other embodiments for such membrane materials can be ion beam apertured polymer films, such as made from PE such as described in "Ion Tracks and Microtechnology - Basic Principles and Applications" edited by R. Spohr and K. Bethge, published by Vieweg, Wiesbaden, Germany 1990.

Other suitable materials are woven polymeric meshes, such as polyamide or polyethylene meshes as available from Verseidag in Geldern-Waldbeck, Germany, or SEFAR in Rüschlikon, Switzerland, for example the type Sefar 03-10/2. Other materials which can be suitable for present applications are hydrophilized wovens, such as known under the designation DRYLOFT ® from Goretex in Newark, DE 19711, USA.

Further, certain non-woven materials are suitable, such as available under the designation CoroGard ® from BBA Corovin, Peine, Germany, can be used, namely if such webs are specially designed towards a relatively narrow pore size distribution, or hydrophilic meltblown nonwovens with resin incorporated surfactant as supplied by Kuraray Co.,Ltd, Osaka, Japan, under the designation PC0015EM-0 having a basis weight of 15 g/m²or PC0030EM-0 having a basis weight of 30 g/m².

For applications with little requirements for flexibility of the members, or where even a certain stiffness is desirable, metal filter meshes of the appropriate pore size can be suitable, such as HIGHFLOW of Haver & Böcker, in Oelde, Germany

The membrane assembly can - in addition to the porous membrane material - have a support element, which as such does not need to contribute to the liquid handling functionality of the assembly, but which provides mechanical support to strengthen the overall structure, or to allow achieving and/or maintaining the desired shape of the assembly.

Such a support element can be a sheet material connecting the ridges of a corrugated structure, or it can be a stiffening agent applied to the ridges of such corrugated structures, thereby reducing the tendency for collapse of such corrugations.

Such a support element can be a sheet like structure, such as being made of or comprising nets, scrims, apertured or reticulated films, wovens, knitted, or nonwoven materials. If such materials envelop the membrane assembly essentially over the entire surface or at least the liquid receiving region, it preferably should have a liquid permeability, which does not significantly hinder the liquid transport, such as by having a permeability which is at least 1000 time, even more preferably 100,000 times the permeability of the membrane material. A method for determining such permeabilities is described in more detail in PCT application US 99/14654.

Such support structures can also be in the shape of stripes, bands, struts or strands, which do not completely envelop the assembly, and thus can be of essentially impermeable material. Such support structure can have an elastic behavior, i.e. can comprise elastomeric material molecules, and can have an isotropic or non-isotropic elastic behavior, such as can be exemplified by net like materials with elastomeric strands extending in one direction, and non-elastomeric ones in the other.

In a further aspect, the present invention relates to methods to create liquid handling members.

In general, methods of making a liquid handling members in the meaning of the present invention have the steps of:
- providing a porous membrane material having a bubble point pressure when having its pores filled with a liquid,
- attaching and hermetically sealing this membrane to a suction device, such that this membrane material separates a first zone outside of the member from a second zone inside the member, whereby the latter zone is connected to a device creating a potential difference, such as a suction device, or a device to create a vacuum,
- such that for a potential differential such as a pressure differential between the first and the second zone, which is below the bubble point pressure of the membrane material, liquid can penetrate through the membrane, but gas cannot.

In addition to these steps, a method according to the present invention has the steps of
- creating a morphology change of said membrane material so as to increase the ratio of the actual surface area to the projected surface area,
- and fixing this morphology change at least for its intended use period.

The following description describes particular ways to create a structure having a high effective surface area.

First, for the selective removal of membrane material, the starting point can be a porous three-dimensional material, such as a foam material having the appropriate pore sizes and pore size distribution. A morphology change to increase the area ratio can then be created by removing certain regions from this material. For example, when referring to the schematic diagram of Fig. 3, the original material (also referred to as precurser) can have the overall thickness H and valleys are created by removing the material until a thickness d in the respective regions results. Such a process is particular useful for materials, where the re-use of the carved out materials can be readily achieved, such as by recycling it into the porous material making process. The fixation of the material would be automatically achieved upon finishing the selective removal.

A further approach to creating membrane assemblies having increased area ratios can be followed by starting from essentially flat membrane materials and by permanently deforming these. Suitable processes are well known as such in the art, such as using vacuum-forming, or hydro-forming technologies (both applied e.g. to films or nonwovens or wovens), or other mechanical stretching processes such as tentering or "ring-rolling" (i.e. stretching between two intermeshing rolls).

An important consideration for this aspect is, that the pore size must be adjusted such that the pore size providing a useful bubble point pressure is achieved after application of such processes, and hence the pore size of the starting material has to be chosen so as to still provide a sufficiently high bubble point pressure after stretching or deformation. This is a much more relevant criterion for the current application as compared to e.g. filtering technology, as in the latter field a quantitative deterioration will generally occur with a few pores being too wide, whilst in the present case the basic functionality can be at risk, if the internal vacuum cannot be maintianed.

Once a material is appropriately formed to provide the appropriate pore size and bubble point pressure, the structure can be fixed by removing the deformation forces, or by changing the temperature under which the deformation was performed. Also, form setting resins can be applied, which can connect various elements and keep them in the shape as formed. Such resins are well known in the art as binder for non-wovens or foam materials.

A preferred way to achieve a liquid handling member having an increase area ratio can be followed when starting from an essentially flat, two-dimensionally extending membrane material. This membrane can be corrugated or undulated or shirred according to conventional techniques, such as described in US-A-3.804.688, US-A-5.753.343; US-A-4.874.457; US-A-3.969.473, US-A-4.239.719.

The fixation of the corrugations can be achieved (as partly described therein) by connecting and attaching the proximal ends of the ridges with a sheet or strip-like support material as described in the above, or by applying themosetting resins to prevent deformation of certain regions of the assembly, such as at the ridges or valleys.

The membrane material and the support material can be affixed to each other in various ways, such by adhesively attaching, or heat-bonding, optionally resin curing / heat setting or entangling such as when knitting or weaving, by sewing, or any other method, as long as the membrane functionality is not lost, and/or the bubble point pressure is not unduly affected. In particular, sharp edges should be avoided, as these can result in deformation of the material, thereby deforming part of the pores, and thus creating pores allowing air or gas to penetrate through during use.

A particularly preferred way to achieve such corrugations is by combining the essentially flat membrane material with a stretched elastomeric support material, such as an open-porous nonwoven, a scrim material, a net or strands or struts or other essentially one-dimensionally extending structures as described in the above, extending perpendicular to the intended direction of the ridges or valleys of the undulations or corrugations.

Once the membrane and the carrier material are in an aligned position, attachment of the two can be achieved by conventional methods like adhesives or thermo-bonding, as described in the above, without unduly damaging the membrane functionality. The attachment can be made in a regular pattern, such as by providing adhesive lines, or by a pattern of regularly arranged bonding points, or by a irregular pattern, such as can be a result of adhesive sprays in certain areas.

Once the attachment is achieved, and the force which extends the elastomeric material is released, the membrane will corrugate or undulate according to the attachment pattern upon relaxation of the elastomeric material.

Alternatively, heat-shrinkable materials can be used instead of elastomeric materials, whereby the membrane is attached to such a heat shrinkeable material in the described attachment pattern, when the heat shrink material is flat but unstretched. The corrugations or undulations will then be formed upon contraction upon heating.

### Test procedures

### Bubble Point Pressure of the membrane material.

The following procedure applies when it is desired to asses the bubble point pressure of a material useful for the present invention.

First, the material is connected with a funnel such as a translucent plastic funnel Catalog # 625 617 20 from Fisher Scientific in Nidderau, Germany and a flexible tubing (inner diameter about 8 mm) such as Masterflex 6404-17 by Norton, distributed by the Barnant Company, Barrington, Illinois 60010 U.S.A. Thereby, the lower end of the tube is left open i.e. not covered by a port region material. The tube should be of sufficient length, i.e. up to 10 m length may be required.

In case the test material is very thin, or fragile, it can be appropriate to support it by a very open support structure (as e.g. a layer of open pore non-woven material) before connecting it with the funnel and the tube. In case the test specimen is not of sufficient size, the funnel may be replaced by a smaller one (e.g. Catalog # 625 616 02 from Fisher Scientific in Nidderau, Germany). If the test specimen is too large size, a representative piece can be cut out so as to fit the funnel.

The testing liquid can be the transported liquid, but for ease of comparison, the testing liquid should be a solution of 0.03% TRITON X-100, such as available from MERCK KGaA, Darmstadt, Germany, under the catalog number 1.08603, in destilled or deionized water, having a surface tension of 33mN/m, when measured according to conventional surface tension methods.

The device is filled with testing liquid by immersing it in a reservoir of sufficient size filled with the testing fluid and by removing the remaining air with a vacuum pump.

Whilst keeping the lower (open) end of the funnel within the liquid in the reservoir, the part of the funnel with the port region is taken out of the liquid. If appropriate - but not necessarily - the funnel with the port region material should remain horizontally aligned.

Whilst slowly continuing to raise the port material above the reservoir, the height is monitored, and it is carefully observed through the funnel or through the port material itself (optionally aided by appropriate lighting) if air bubbles start to enter through the material into the inner of the funnel. At this point, the height above the reservoir is registered to be the bubble point height.

From this height H the bubble point pressure bubble point pressure is calculated as: *BPP* = ρ·*g*·*H* with the liquid density ρ, gravity constant g (*g* ≈ 9.81 m/s²).

In particular for bubble point pressures exceeding about 50kPa, an alternative determination can be used, such as commonly used for assessing bubble point pressures for membranes used in filtration systems.
Therein, the wetted membrane is separating two gas filled chambers, when one is set under an increased gas pressure (such as an air pressure), and the point is registered when the first air bubbles "break through". Alternatively, the PMI permeater or porosity meter, as described in the test method section of PCT application US 98/13497, and incorporated herein by reference, can be used for the bubble point pressure determination.

### Bubble point pressure of the liquid transport member

For measuring the bubble point pressure of a liquid transport member (instead of a membrane material), the following procedure can be followed.

First, the member is activated as described hereinabove for the membrane material. A part of a port region under evaluation is connected to a vacuum pump connected by a tightly sealed tube (such as with suitable adhesive).
Care must be taken, that only a part of the port region is connected, and a further part of the region next to the one covered with the tube is still uncovered and in contact with ambient air. The vacuum pump should allow to set various pressures P_{vac}, increasing from atmospheric pressure Pₐₜₘ to about 100 kPa. The set up (often integral with the pump) should allow monitoring the pressure differential to the ambient air (Δp = pₐₜₘ - p_{vac}) and of the gas flow.

Then, the pump is started to create a light vacuum, which is increased during the test in a stepwise operation. The amount of pressure increase will depend on the desired accuracy, with typical values of 0.1 kPa providing acceptable results.

At each level, the flow will be monitored over time, and directly after the increase of Δp, the flow will increase primarily because of removing gas from the tubing between the pump and the membrane. This flow will however, rather quickly level off, and upon establishing an equilibrium Δp, the flow will essentially stop. This is typically reached after about 3 minutes.

This step change increase is continued up to the break through point, which can be observed by the gas flow not decreasing after the step change of the pressure, but remaining after reaching an equilibrium level essentially constant over time.

The pressure Δp one step prior to this situation is the bubble point pressure of the liquid transport member.

For materials having bubble point pressures in excess of about 90 kPa, it will be advisable or necessary to increase the ambient pressure surrounding the test specimen by a constant and monitored degree, which is then added to Δp as monitored.

### Bulk Softness

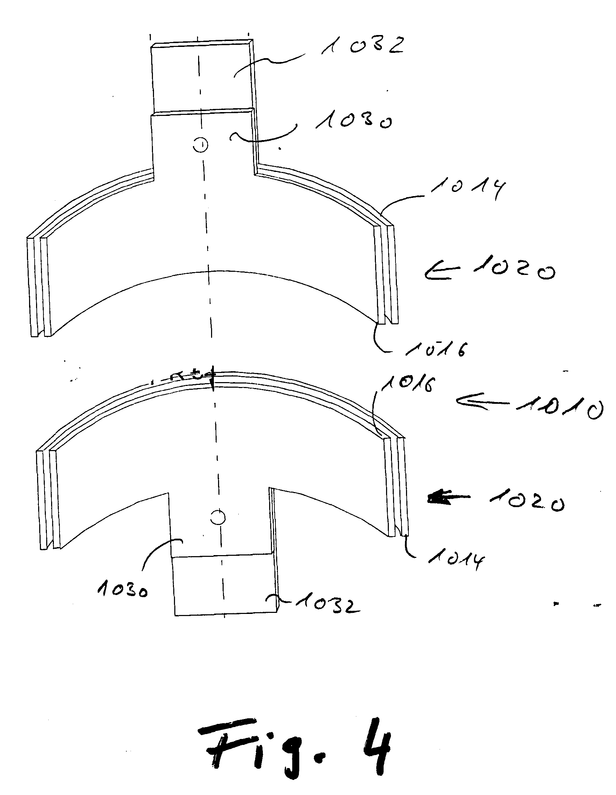

This method is intended to measure individual materials as well as structures comprising these materials. The method uses a tensile tester in compressive mode and a sample holder (Figure 4) to measure the buckling force for a sample.

A suitable tensile tester is available from Zwick Company of Ulm, Germany as a Zwick Material Tester type 144560.

The sample holder (1010) for this test is shown in Figure 4. As can be seen therein the sample is held between two curvilinear plates (1020) that have tabs (1030, 1032) 30 mm wide that extend upward 20 mm (front element, 1030) and 55 mm (rear element, 1032) so as to enable insertion of the sample holder (1010) into the jaws of the tensile tester. Readily the curvature of the outer element of the holder (1014) has a radius of 59 mm±1 mm with an arc length of 150 mm and the inner element (1016) has a radius of 54 mm±1 mm with an arc length of 140 mm The equipment is designed to test various material thickness from 1 mm up to 10 mm. As will be recognized, sample holders of this type are necessary for both the upper and lower jaws of the tensile tester.

Prior to testing a sample is conditioned under controlled conditions (50% RH, 25°C) for at least two hours. The sample is cut to 60 mm by 150 mm (±2 mm per dimension). The sample dimensions, short side vs. long side, should be consistent with the bending axis orientation for which the test is executed, and can be aligned with the intended use in a finished product, whereby the y-axis generally corresponds to the left-right orientation of the user, and generally to the width dimension of the article, and the x-axis being perpendicular thereto. The operation is as follows:
1. The tensile tester is calibrated (in compressive mode) according to the manufacturer's instructions.
2. The compression rate is set to 200 mm/minute and the crosshead stop point to 30 mm.
3. A sample is inserted into the sample holder to a depth of 7 mm ±1 mm for each clamp set.
4. The tensile tester jaw separation is set so that the unconstrained portion of the sample is smooth and unbuckled. This corresponds to a spacing between the upper and lower portions of the sample holder of 46 mm.
5. The sample/sample holder assembly is inserted into the jaws of the tensile tester.
6. The tensile tester is operated in compressive mode to record a force/compression curve for each sample.
7. The buckling force for each sample is recorded, which is the force required to cause the sample to initially begin to bend. It is the initial peak force that is seen on the force compression curve before a relatively constant force plateau that is a measure of the bending resistance of the sample (bending force) and is expressed in Newton (N).
8. Repeat steps 5 to 7 for at least 5 samples for each structure tested and report the average and standard deviation of the buckling force.

## Claims

1. A liquid handling member comprising an first zone,
and a second zone connected to a suction device,
said first zone and said second zone being separated by a porous membrane assembly comprising a membrane material having a actual surface area along its surface contours,
wherein said membrane assembly has a projected surface area
projected on an surface generally aligned with the member surface during its intended use
whereby said membrane assembly is capable of maintaining a pressure differential between the second zone and the first zone without permitting air to penetrate from said first zone to said second zone.
characterized in that
said membrane material has an actual surface area which is
at least 2, preferably 8, more preferably 20, even more preferably 40 times the area of said projected surface of said membrane assembly,
and which is
not more than 200, preferably not more than 100, and even more preferably not more than 80 times the area of said projected surface of said membrane assembly
when measured without a load applied to the member.

2. A liquid handling member according to claim 1, wherein
said actual surface area of said membrane material is at least 2 , preferably 8, more preferably 20, even more preferably 40 times the area of said projected surface of said membrane assembly,
and not more than 200, preferably not more than 100, and even more preferably not more than 80 times the area of said projected surface of said membrane assembly, when said member is submitted to an external load pressure of at least about 2070 Pa (0.3 psi), preferably when submitted to a pressure exceeding about 4800 Pa (0.7 psi), or even about 9650 Pa (1.4 psi), when applied perpendicular to said projected surface of said membrane assembly and related to said projected surface area.

3. A liquid handling member according to claim 1 or 2
wherein said membrane assembly has two enveloping surfaces generally parallel to said projected surface area, which define a Cartesian coordinate system with a x (length), y (width), and z (thickness) direction,
and are arranged at a distance H from each other, whereby H is greater than the material thickness of the membrane,
which is generally aligned with the flow path of the liquid penetrating through the pores of said membrane material.

4. A liquid handling member according to any of claims 1 to 3
wherein said membrane assembly has a three-dimensionally shaped morphology having repeating geometric cells defined by repeating geometric pattern of cross-sectional view through said membrane assembly.

5. A liquid handling member according to claim 4, wherein said
repeating geometric cells are arranged in checkerboard pattern.

6. A liquid handling member according to claim 4, wherein said
repeating geometric cells are arranged in a row pattern.

7. A liquid handling member according to claim 4,
wherein said repeating geometric cell of said membrane assembly is in the form of corrugations, pleats, or folds of a sheet-like membrane material having a pore size r and a material sheet thickness d,

8. A liquid handling member according to claim 7,
having more than 0.3 corrugation, pleats or folds per centimeter.

9. A liquid handling member according to claim 7,
having less than 20 corrugations, pleats or folds per centimeter.

10. A liquid handling member according to claim 7,
having corrugations, pleats or folds having a height of more than 0.05 mm.

11. A liquid handling member according to claim 7,
having corrugations, pleats or folds having a height of less than 30 mm.

12. A liquid handling member according to claim 7,
wherein said corrugations, pleats or folds have repeating cross-sectional pattern.

13. A liquid handling member according to any of claims 7 to 12,
wherein the repeating pattern is circular, sinusoidal, parabolic or elliptic.

14. A liquid handling member according to any of claims 7 to 13, wherein
said geometric cell has a characteristic height H, and repeating unit width L, and wherein the ratio (L²/H) is at least 10 times, preferably 20 time, more preferably 50 times the ratio of (r²/d).

15. A liquid handling member according to claim 14,
maintaining the ratio (L²/H) of at least 10 times, preferably 20 time, more preferably 50 times the ratio of (r²/d).
under an external load pressure of at about 2070 Pa (0.3 psi), preferably when submitted to a pressure exceeding about 4800 Pa (0.7 psi), or even about 9650 Pa (1.4 psi) applied along the height of said corrugations and related to the area of the projected surface area.

16. A liquid handling member according to any of the preceding claims,
wherein said membrane assembly further comprises a support structure for maintaining said geometric shape.

17. A liquid handling member according to claim 16, wherein said support structure is generally aligned with an enveloping surface of said membrane assembly.

18. A liquid handling member according to claim 16 or 17
wherein said support structure has a liquid permeability of 1000 times preferably 100.000 times the permeability of said membrane material.

19. A liquid handling member according to any of claims 16 to 18,
wherein said membrane assembly is corrugated, pleated of folded, and wherein said support structure is arranged to fix the corrugations, pleats or folds.

20. A liquid handling member according to claim 16
wherein said membrane and said support structure are affixed to each other by a fixation means, preferably by adhesive or melt fusion.

21. A liquid handling member according to claim 20
wherein said fixations means is applied in a non-continuous bonding pattern, preferably point bonding.

22. A liquid handling member according to any of claims 16 to 21,
wherein said support structure comprises elastomeric material.

23. A liquid handling member according to claim 22,
wherein said elastomeric material is a net, scrim, woven, knitted, or nonwoven material.

24. A liquid handling member according to claim 22,
wherein said elastomeric material is an elastomeric film.

25. A liquid handling member according to any of claims 22 to 24
wherein said elastic material is in the form of strands or struts.

26. A liquid handling member according to any of claims 22 to 25.
wherein said elastomeric material is an essentially two-dimensional web or composite of strands or struts, which has non-isotropic elastic properties and has a higher elasticity in one direction than in the one perpendicular thereto.

27. A liquid handling member according to any of the preceding claims,
having a buckling force as measured according to the Bulk Softness method of less than 10 N, preferably less than 5 N, more preferably less than 3 N, most preferably less than 1 N.

28. A method for making a liquid handling member, comprising the steps of
providing a porous membrane material having a bubble point pressure;
creating a morphology change of said membrane material so as to increase the projected versus actual surface for the membrane region;
fixing said morphology change to form a membrane assembly,
attaching and hermetically sealing said membrane assembly to a suction device, such that said membrane material separates a first zone (outside of the member) from a second zone inside the member, which is connected to said suction device,
such that for a pressure differential between the first and the second zones, which is below the bubble point pressure of the membrane material, liquid can penetrate through the membrane but gas not.

29. A method for making a liquid handling member according to claim 28,
wherein said morphology change is a selective removal of membrane material from a (bulk, precursor) membrane material.

30. A method for making a liquid handling member according to claim 28,
wherein said morphology change is a plastic deformation of a plastically deformable membrane material.

31. A method for making a liquid handling member according to claim 30, wherein said membrane has an bubble point pressure after plastic deformation which is more than 0.9 times the bubble point pressure of the membrane material prior to deformation.

32. A method for making a liquid handling member according to any of claims 28 to 31 wherein said membrane is an essentially two-dimensional sheet material.

33. A method for making a liquid handling member according to claim 32, wherein said web material is deformed by stretching.

34. A method for making a liquid handling member according to claim 33, wherein said stretching is one dimensional, preferably by tentering, nip roll stretching with varying roll speeds, or by feeding the material between two intermeshing rolls.

35. A method for making a liquid handling member according to claim 33, wherein said stretching is achieved by hydroforming, or vacuum forming, or blow molding.

36. A method for making a liquid handling member according to any of claims 28 to 35, wherein said morphology change is the arrangement of the membrane material in corrugations, pleats or folds.

37. A method for making a liquid handling member according to claim 36, wherein said arrangement in pleats or folds or corrugations is created by feeding said membrane material as a roll-stock over a shaped roll under no sideways constraint, such that the arrangement of pleats or corrugations is achieved by narrowing the two longitudinal edges of the web to each other.

38. A method for making a liquid handling member according to claim 37 whereby said arrangement in pleats or folds or corrugations is in transverse direction of the roll-stock web.

39. A method for making a liquid handling member according to any of claims 28 to 38 whereby said step of fixing the morphology change further comprises the steps of providing an essentially flat support material, which is permeable to liquids, combining said support material with said pleated or corrugated membrane material, attaching said flat support material with said pleated or corrugated membrane material.

40. A method for making a liquid handling member according to claim 39, whereby said creation of morphology change and the fixation thereof comprise the steps of
providing an elastically extensible, liquid permeable support material;
stretching said support material;
attaching said support material in selected surface regions to said essentially flat, membrane material;
releasing the stretch of said stretched support material,
thereby creating shirring of the membrane material.

41. A method for making a liquid handling member according to any of claims 28 to 40, whereby said steps of fixing said morphology change and sealing and hermetically attaching said assembly are executed simultaneously.
